# EUROPEAN PATENT SPECIFICATION

(11) **EP 0 725 825 B1**
(45) Date of publication and mention of the grant of the patent: **21.02.2001**
(21) Application number: 94927602.6
(22) Date of filing: 12.09.1994
(51) Int. Cl.: C12N 15/48, C07K 14/16, A61K 39/21, A61K 39/395, C07K 16/10

(54) **PEPTIDES THAT ELICIT NEUTRALIZING ANTIBODIES AGAINST GENETICALLY DIVERGENT HIV-1 STRAINS**
PEPTIDE, DIE NEUTRALISIERENDE ANTIKÖRPER GEGEN GENETISCH DIVERGIERENDE HIV-1 STÄMME, INDUZIEREN
PEPTIDES QUI PRODUISENT DES ANTICORPS NEUTRALISANTS CONTRE DES SOUCHES DE VIH-1 GENETIQUEMENT DIVERGENTES

(30) Priority: 11.09.1993 EP 93114631
(43) Date of publication of application: 14.08.1996
(73) Proprietor: Polymun Scientific Immunbiologische Forschung GmbH, 1180 Wien (AT)
(72) Inventor: KATINGER, Hermann, A-1190 Wien (AT); MUSTER, Thomas, A-1180 Wien (AT)
(74) Representative: Büchel, Kurt F., Dr.
(86) International application number: EP9403039
(87) International publication number: WO9507354

(56) References cited:
- EP-A- 0 570 357
- AIDS RESEARCH AND HUMAN RETROVIRUSES, vol.9, no.7, July 1993 pages 581 - 587 ALLAWAY ET AL. 'Synergistic inhibition of HIV-1 envelope-mediated cell fusion by CD4-based molecules in combination with antibodies to gp120 or gp41'
- SCIENCE., vol.250, no.4987, 14 December 1990, LANCASTER, PA US pages 1590 - 1593 JAVAHERIAN ET AL. 'Broadly neutralising antibodies elicited by the hypervariable neutralizing domain of HIV-1'
- NATURE., vol.339, no.6223, 1 June 1989, LONDON GB pages 385 - 388 EVANS ET AL. 'An engineered poliovirus chimaera elicits broadly reactive HIV-1 neutralizing antibodies'
- AIDS, vol.7, no.2, February 1993, LONDON, UK. pages 167 - 174 VANINI ET AL. 'Discrete regions of HIV-1 gp41 defined by syncytia-inhibiting affinity-purified human antibodies'
- PROCEEDINGS OF THE NATIONAL ACADEMY OF SCIENCES OF USA., vol.89, January 1992, WASHINGTON US pages 461 - 465 BROLIDEN ET AL. 'Identification of human neutralizing-inducing regions of the human immunodeficiency virus type 1 envelope glycoproteins'
- JOURNAL OF VIROLOGY, vol.67, no.11, November 1993 pages 6642 - 6647 MUSTER ET AL. 'A conserved neutralizing epitope on gp41 of human immunideficiency virus type 1'

## Description

The present invention relates to peptides which elicit neutralizing antibodies against different strains and clinical isolates of HIV-1, as well as to the antibodies elicited therewith, and also to the preparation of peptide-carrier combinations that efficiently present these peptides to the immune system. More particularly, this invention relates to the development of a vaccine against HIV-1.

The acquired immunodeficiency syndrome (AIDS) is the late stage clinical manifestation of a long term persistent infection with human immunodeficiency virus type 1 (HIV-1). Immune responses directed at the virus and at virus-infected cells during the persistent infection usually fail to mediate the resolution of the infection. Vaccines may elicit immune responses that can prevent the establishment of a persistent infection or even prevent the progression to AIDS. Most vaccine strategies against HIV-1 are directed at its surface glycoprotein gp160 which is made up of gp120 and gp41 and is responsible for binding the virus to the cellular receptor CD4 and for triggering subsequent fusion activity.

However, in context with gp160, several phenomena were observed that argue against the use of whole gp160 or gp120 as an immunogen. In vitro experiments show that synergism between gp120 and gp120-specific antibodies block human T-cell activation (Mittler et al., Science (1989)245:1380). In addition, a number of antigenic domains on gp160 are known to induce antibodies that enhance HIV-1 infection (Jiang et al., J. Exp. Med. (1991) 174:1557).

The use of synthetic peptides as immunogens offers a number of advantages. The antibodies elicited by synthetic peptides have a predetermined specificity, and in the case of viruses, they can be selected to represent structures on the surface of virions. The synthetic polypeptides are interesting also in that they can induce antibody responses not seen under normal conditions. For example, it is possible to induce neutralizing antibodies that have a broader reactivity than antibodies induced by whole proteins (Green et al., Cell (1982) 28:477).

In addition, a peptide containing part of the V3 loop of gp120 from the HIV-1 isolate HIV-1 IIIB was shown to induce antibodies that protected chimpanzees against virus challenge with the same HIV-1 isolate (Emini et al., Nature (1992) 355:728).

Because synthetic peptides themselves have poor immunogenicity, they have to be coupled to molecules that provide an adjuvant effect such as tetanus toxoid or keyhole limpet hemocyan (Bittle et al., Nature (1982) 298:30). Another possibility is to clone small peptides as fusion proteins with glutathione S-transferase of Schistosoma japonicum (Fikrig et al., Science (1990) 250:553).

Furthermore, viruses such as vaccinia, polio or influenza can be used as vectors for immunogens. Rabbits inoculated with recombinant vaccinia virus containing sequences from hepatitis B surface antigen (HBsAg), herpes simplex virus glycoprotein D, and influenza virus hemagglutinin produced antibodies to all three foreign antigens (Perkus et al., Science (1985) 229:981). Furthermore, a chimeric polio virus that expressed an epitope from gp41 of HIV-1 successfully induced neutralizing antibodies against HIV-1 in rabbits (Evans et al., Nature (1989) 339:385).

Since recently, it is also possible to change the genome of influenza virus by in vitro mutagenesis (Enami et al., Proc. Natl. Acad. Sci. USA (1990) 87:3802). By means of this technique it was possible to engineer a stable attenuated influenza A virus (Muster et al., Proc. Natl. Acad. Sci. USA (1991) 88:5177).

An advantage of influenza virus in this context is the availability of many variants so that repeated vaccination may be possible. Furthermore, influenza virus induces strong secretory and cellular immune responses, which may be advantageous for an anti-HIV-1 vaccine approach.

It is the object of the instant invention to provide peptide sequences, that are only minimally immunogenic in the context of the whole gp160, and which peptide sequences can be used to elicit antibodies that show neutralizing activity against different strains and/or clinical isolates of HIV-1 and/or that inhibit the fusion of cells caused by HIV-1 in mammals.

It is a further object of the invention to provide strategies to elicit secretory antibodies secreted from mucosal surfaces and directed at HIV-1 virus and virus infected cells. It was presumed according to the invention that eliciting anti-HIV-1 IgA antibodies in mucosal tissues will provide a powerful tool especially in the prophylaxis and prevention of potentially endangered individuals from HIV-1 infection, since many viral infections including HIV-1 are transmitted via mucosal surfaces of the respiratory, gastrointestinal and genital tract.

The objects are achieved by preparing small peptides with seven or eight amino acids obtained either by chemical synthesis or by microbiological methods, the peptides preferably being derived from nucleic acid sequences coding for Glu Leu Asp Lys Trp Ala Ser (= ELDKWAS in the one-letter-code), Leu Glu Leu Asp Lys Trp Ala Ser (LELDKWAS), Glu Leu Asn Lys Trp Ala Ser (ELNKWAS), Leu Glu Leu Asn Lys Trp Ala Ser (LELNKWAS), Glu Leu Asp Asn Trp Ala Ser (ELDNWAS) or Leu Glu Leu Asp Asn Trp Ala Ser (LELDNWAS).

At least one, preferably all, of the above six amino acid sequences (hereinafter referred to as "said six AAS") may then efficiently be presented to the immune system in order to induce formation and release of antibodies that show neutralizing activity against HIV-1 strains and/or which can inhibit cell fusion caused by these viruses.

It is a major goal of the instant invention to provide a promising anti-HIV-1 vaccine based on a formulation comprising at least one, preferably a mixture of all six, of the above peptides. Special features and advantageous embodiments of the invention are described below.

In a preferred embodiment of the invention, the amino acid sequence Glu Leu Asp Lys Trp Ala (ELDKWA) defining the epitope sequence of the human monoclonal antibody 2F5 (obtained from hybridoma cell line 2F5, PHLS deposit Nr.90091704; deposited 09/17/90) is connected with a Ser or both a Leu and Ser - which are located adjacent to the ELDKWA Sequence on gp41 defining the 2F5-epitope - thereby establishing the peptide sequences Glu Leu Asp Lys Trp Ala Ser (ELDKWAS) and Leu Glu Leu Asp Lys Trp Ala Ser (LELDKWAS) respectively, and inserted into a carrier molecule, namely the antigenic site B of the HA of influenza virus.

The modified "chimeric" HA fusion protein, carrying the foreign amino acid sequence ELDKWAS or LELDKWAS, very efficiently elicits antibodies directed at the presented ELDKWAS or LELDKWAS sequence upon application, preferably in the form of an injection, to the immune system of mammals, and humans in particular. The antibodies obtained by this method show neutralizing activity against different strains and/or clinical isolates of HIV-1.

It has, however, turned out that due to the known polymorphism of the HIV-1 virus mutations can occur even within the highly conserved (L)ELDKWAS amino acid sequence of gp41, which corresponds to amino acid 661-668 (LELDKWAS) or 662-668 (ELDKWAS) of HIV-1 isolate BH10. The nucleotide and amino acid numbering used throughout this specification corresponds to that of gp160 of HIV-1 isolate BH10 as used in the Los Alamos data base (Myers et al., Data Base Human Retrovirus and Aids). The mutations particularly affected the Asp (D) and/or Lys (K) amino acids in the centre of said sequence, thereby reducing the virus' susceptibility to getting neutralized by antibodies carrying the 2F5 epitope. This undesired kind of viral escape mechanism could, however, successfully be overcome by using those peptides according to the instant invention, wherein either Asp (D) or adjacent Lys (K) is replaced with asparagine (N).

At least one, preferably a mixture of all six, of said six peptides may be used for the manufacture of a pharmaceutical to elicit antibodies that show neutralizing activity against different strains and/or clinical isolates of HIV-1 and/or that inhibit the fusion of cells caused by HIV-1.

In another embodiment, the same peptides may be used either each peptide alone or in a mixture with at least one other of said peptides, to manufacture a pharmaceutical to induce anti-HIV-1 IgA antibodies secreted from mucosal surfaces upon, preferably intranasal, application to mammals.

In a further embodiment of the invention, at least one of said six peptides is linked to a carrier. Such carrier can, for instance, be a virus, preferably in its attenuated and/or recombinant form. Advantageously, influenza virus, baculovirus or vaccinia virus may be used. Linking the small peptides to a carrier such as, for instance, a viral protein or a complete virus enhances the efficacy of antibody induction upon presentation of such chimeric peptide-carrier combinations or chimeric fusion proteins to the immune system.

It is particularly advantageous, to use each of the above peptides as a fusion protein with a viral protein as the carrier, wherein at least one of the amino acid sequences according to the invention substitutes at least one part of the viral carrier protein or is inserted into at least one of the antigenic sites of the viral protein. This feature, therefore, represents an important embodiment. In a preferred embodiment, said viral protein is the hemagglutinin (HA) of influenza virus, the neuraminidase (NA) of influenza virus or the surface antigen of hepatitis B virus. In another embodiment, it may be derived from a - preferably recombinant - baculovirus or vaccinia virus.

The instant invention also relates to the use of any of the above identified peptides consisting of seven or eight amino acids and/or peptide-carrier combinations or fusion proteins for the manufacture of a pharmaceutical to elicit or induce antibodies that show neutralizing activity against different strains and/or clinical isolates of HIV-1 and/or that inhibit the fusion of cells caused by HIV-1. More particularly, it is intended to provide an effective vaccine based on at least one, preferably a mixture of all six, of said peptides and/or said six peptides linked to a carrier, for the prophylactic treatment of HIV-1 endangered individuals and/or the therapeutic treatment of HIV-1 infected patients, especially to prevent the progression of the infection to AIDS.

In a further preferred embodiment, said six peptides and/or said six peptides linked to a carrier are used to prepare and manufacture pharmaceutical formulations that - upon application to the mammalian immune system - lead to the induction of anti-HIV-1 IgA antibodies secreted from mucosal surfaces of the animal or human patients. Intranasal application is preferred in this case.

Consequently, said six peptides and/or peptide-carrier combinations of the instant invention may also be used for the manufacture of a pharmaceutical for the prophylaxis and prevention of endangered individuals from HIV-1 infection.

It appears, that this special feature of the instant invention may give rise to the hope that by way of eliciting anti-HIV-1 specific secretory IgA antibodies in mucosal surfaces according to the instant invention a medical tool is provided that effectively attacks the viral invaders already at the very first stage of their entrance to the mammalian body. It is believed that the prior art methods to treat HIV-1 infected patients at least partly fail to defend the disease because the virus is chased primarily in the blood stream and at a rather late stage, i.e. after widespread distribution in the humoral system.
The instant invention further relates to an antibody showing HIV-1 neutralizing activity and being capable of preventing fusion of cells caused by HIV-1, characterized in that it is elicited by one of the said six peptides.

In a preferred embodiment, the said antibody is a secretory antibody, preferably an anti-HIV-1 IgA antibody, and advantageously being secreted by mucosal surfaces.

Furthermore, the instant invention also refers to a process for the manufacture of anyone of said six peptides by either microbiological or chemical methods.

In one embodiment said six peptides are chemically synthesized following standard biochemical procedures, preferably using an Applied Biosystems 431A Peptide Synthesizer.

In another embodiment, said amino acid sequences are obtained by a microbiological process comprising the steps of inserting a nucleotide sequence selected from the group consisting of
a) a nucleotide sequence corresponding to one of said six peptides;
b) a nucleotide sequence hybridizing to one of the nucleotide sequences under a);
c) a nucleotide sequence deduced from one of the nucleotide sequences under a) by degeneration;
into the genome of a host organism, carrying out expression of the genome using standard microbiological cultivation techniques, and isolating at least one, preferably a multitude, of said peptides.

Chimeric fusion proteins or peptide-carrier combinations may be manufactured according to an embodiment comprising linking an amino acid sequence selected from the group consisting of said six peptides, to a carrier, preferably a virus or viral protein, by chemical or microbiological methods.

In a preferred embodiment, the process for the manufacture of the said six peptides linked to a carrier, preferably a virus or viral protein, is microbiological and comprises the steps of linking a nucleotide sequence selected from the group consisting of
a) a nucleotide sequence corresponding to one of the amino acid sequences of the said six peptides;
b) a nucleotide sequence hybridizing to one of the nucleotide sequences under a);
c) a nucleotide sequence deduced from one of the nucleotide sequences under a) by degeneration;
with a nucleotide sequence corresponding to an amino acid sequence of the carrier, transferring the linked nucleotide sequences to a host organism, carrying out expression of the linked sequence using standard microbiological techniques, and isolating at least one, preferably a multitude, of said peptides linked to a carrier.

In a further embodiment, the process for the manufacture of said six peptides linked to a carrier, preferably a virus or viral protein, is characterized in that at least one of the nucleotide sequences selected from the group consisting of
a) a nucleotide sequence corresponding to one of the amino acid sequences of said six peptides;
b) a nucleotide sequence hybridizing to one of the nucleotide sequences under a);
c) a nucleotide sequence deduced from one of the nucleotide sequences under a) by degeneration;
is linked with a nucleotide sequence corresponding to an amino acid sequence of a viral protein as the carrier, thereby substituting at least part of the nucleotide sequence corresponding to the amino acid sequence of the viral protein or being inserted into at least one site of said sequence corresponding to an antigenic site of the viral protein.

In a specific embodiment, the applied viral protein is selected from the group consisting of the hemagglutinin of influenza virus, neuraminidase of influenza virus and the surface antigen of hepatitis B virus, and in another characteristic embodiment, an influenza virus, baculovirus or vaccinia virus - each of them preferably in a recombinant form - is used as the host organism.

Insertion of the sequences of said six peptides into the antigenic site B of the HA of influenza strains other than WSN, which strain is mainly referred to in the following examples, will equally lead to fusion proteins and/or chimeric viruses that elicit neutralizing anti-HIV-1 antibodies and prevent HIV-1-driven cell fusion.

Also, introduction of these sequences into other sites of the HA of influenza virus or into the neuraminidase (NA) of influenza virus leads to peptide-carrier fusion proteins and/or chimeric viruses that elicit neutralizing anti-HIV antibodies and prevent HIV-driven cell fusion.

The present invention will be further explained and demonstrated in the following examples, which do not limit in any respect the scope of the present invention.

### Example 1: Epitope mapping

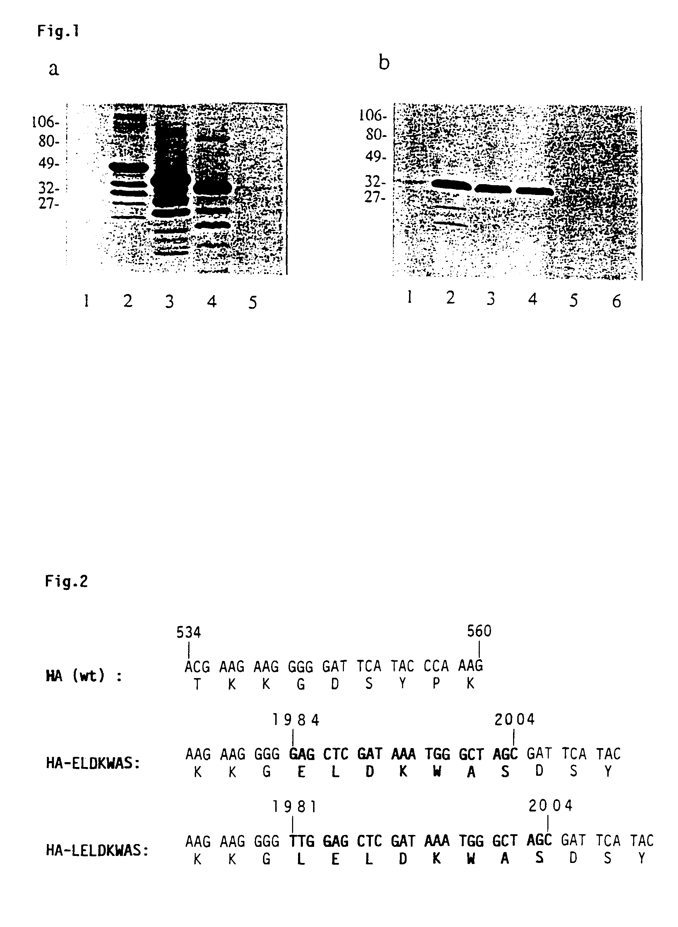

The epitope of the monoclonal antibody 2F5 was identified by immunoblotting using a method described by Towbin et al., Proc. Natl. Acad. Sci. USA (1979)76:4350. Peptides of overlapping fragments of gp41 of HIV-1 isolate BH10 were cloned as fusion peptides with glutathione S-transferase. The different fusion peptides were obtained through hybridization of gp41 corresponding oligonucleotides which were cloned between the Bam HI and the Eco RI site of the plasmid pGEX-2T (Pharmacia). The recombinant plasmids were transformed into the E. coli strain DH5 and expression of the fusion proteins was induced with isopropylthiogalactoside (IPTG). The E. coli extract was then purified with glutathione-sepharose 4B columns, loaded on sodium dodecylsulfate-polyacrylamide gels, separated by electrophoresis, and protein expression was analyzed by silver staining and immunoblotting (Fig.1). The data from the immunoblotting show that the epitope of human monoclonal antibody 2F5 comprises the amino acid sequence ELDKWA corresponding to amino acids 662-667 of gp160.
- Fig. 1: illustrates the specificity of human monoclonal antibody 2F5 (see Example 1).
- Fig. 2: illustrates the construction of chimeric hemagglutinins that carry the 2F5-epitope sequence (see Example 2)
- Fig. 3: illustrates ELISA titers of antisera of mice immunized with the chimeric influenza viruses (see Example 3)
- Fig. 4: illustrates ELISA titers of mice immunized with cells infected with the recombinant baculoviruses (see Example 5).

Fig.1 shows immunoblots of fusion peptides with overlapping fragments of gp160 of HIV-1 (isolate BH10). In contrast to constructs that comprise amino acids 597 to 677 (lane 2), 634 to 677 (lane 3) and 648 to 677 (lane 4) which were reactive with the antibody 2F5, a fusion peptide comprising amino acids 667 to 677 (lane 5) did not show a positive reaction.

This was the first indication that the epitope of the monoclonal antibody 2F5 is formed by amino acids within the sequence from position 648 to 667 of gp160. Based on these results, overlapping 6-mer peptides of this region were fused with the glutathione S-transferase.

As shown in Fig.lb, the peptide containing the amino acid sequence GLU LEU ASP LYS TRP ALA (amino acids 662-667, lane 2) was highly reactive with the antibody 2F5 whereas for peptides containing the aminoacid sequence LEU ASP LYS TRP ALA SER (LDKWAS, amino acids 663-668, lane 3) or ASP LYS TRP ALA SER LEU (DKWASL, amino acids 664-669, lane 4) reactivity with the monoclonal antibody was reduced. A peptide containing aminoacid sequence LEU GLU LEU ASP LYS TRP (LELDKW, amino acids 661-666, lane 1) showed no significant reactivity. These data show that the epitope of the monoclonal antibody comprises the amino acid sequence GLU LEU ASP LYS TRP ALA (ELDKWA) that corresponds to amino acids 662-667 on gp160 of the HIV-1 BH10 isolate. In this context, both a synthetic peptide corresponding to this epitope sequence and a fusion protein containing this sequence were able to inhibit neutralization mediated by the 2F5 antibody.

### Example 2: Construction of plasmids and the chimeric influenza viruses

All genetic manipulations used were done according to standard procedures described by Sambrook et al.,(1989) in "Molecular Cloning", A Laboratory Manual, Cold Spring Harbour Laboratory Press, New York. As illustrated in Fig.2, the gp41-sequences Leu Glu Leu Asp Lys Trp Ala Ser (LELDKWAS) or Glu Leu Asp Lys Trp Ala Ser (ELDKWAS) were inserted into the antigenic site B of the HA of influenza WSN virus. The plasmids pHA-ELDKWAS and pHA-LELDKWAS were constructed by replacing the Pst I-Hind III fragment of plasmid pT3/WSN-HAm1 which is described by Li et al.(Proc. Natl. Acad. Sci. USA 90, 5214-5218;1993) with a PCR product which was obtained by using pT3/WSN-HAm1 as a template and sense and antisense primers derived from influenza virus WSN HAm1, whereby the antisense primer further contained a 21 or 24 nucleotide insertion corresponding to gp160 positions 1981 or 1984 to 2004. The plasmids pHA-ELNKWAS, pHA-LELNKWAS, pHA-ELDNWAS and pHA-LELDNWAS were prepared in the same way.

The sequence of the WSN HA is provided by Hiti et al. (J.Virol.41,730-734,1982) and the sequence of gp160 by the Swissprot database entry ENV$HIV10. Transfection of RNA derived from this plasmid into MDBK cells and selection and preparation of chimeric viruses was done according to Enami et al. (Proc.Natl.Acad. Sci. 87,3802-3805,1990) with modifications described by Enami and Palese (J.Virol.65, 2711-2713, 1991).

### Example 3: Immunization and antibody response of mice immunized with the chimeric influenza viruses

OF-1 mice were immunized with the chimeric influenza-ELDKWAS virus mice (M1, M2, M3, M4) or influenza-L ELDKWAS virus (mouse M5). Mice were first immunized with 10² TCID₅₀ intranasally (i.n.) followed by an i.n. booster immunization with 5x10⁵ TCID₅₀ after 6 weeks, an intraperitoneal (i.p.) immunization with 20µg sucrose-purified live virus after 3 weeks and a final boost with 20µg of SDS-denatured virus in incomplete Freunds adjuvant after 3 more weeks intervals. For intranasal immunization, mice were under ether anesthesia. For the wild-type WSN control virus (wt 1, wt 2), the same protocol was used. Mice were bled 12 days after the final boost, antisera were inactivated 1h at 56°C and ELISA titers and neutralizing activity of antisera was determined.

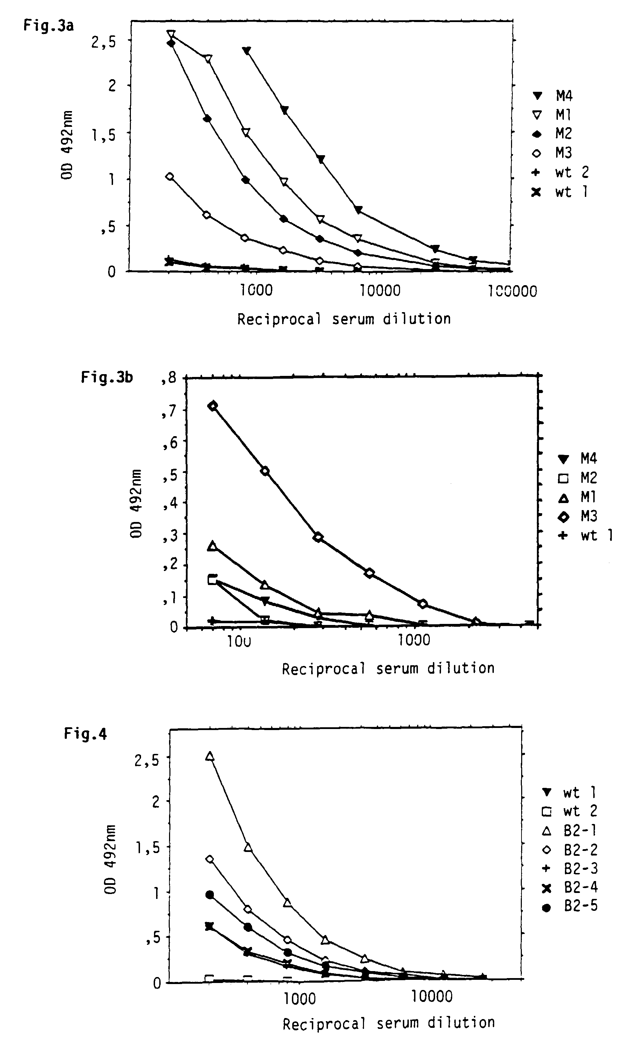

Fig.3A shows the binding of influenza-ELDKWAS antisera to a glutathione S-transferase (GST) fusion peptide containing the sequence ELDKWA on its C-terminus. The sequence was determined using ELISA. 96-well microtitre plates were coated with the GST-ELDKWA fusion peptide at 4µg/ml (100µl/well) in carbonate buffer, pH 9,6 for 4h at room temperature. Plates were then washed with PBS/0.05% Tween. Antiserum diluted in PBS/ 1% BSA/0.05% Tween was added and incubated for 1h at RT. After washing, antibodies were detected by incubation with a goat anti-mouse IgG γ-chain antibody, conjugated with horse radish peroxidase. The plates were stained using o-phenylenediamine-dihydrochloride as a substrate. The reaction was stopped with 2.5 M H₂SO₄ and the plates were measured (measure wavelength 492 nm, reference wavelength 620 nm).

Fig 3B: The same procedure as described in Fig.3A was followed except that for detection of antibodies a goat anti-mouse IgA antibody, conjugated with horse-radish peroxidase was used. The neutralizing activity of the antisera was determined by syncytia inhibition assays. The reciprocal serum dilutions that inhibit syncytia formation by 50% (EC₅₀) are shown in Table 1. Antisera from mice M1 - M3 neutralized the entire test panel at various serum dilutions. Antisera M4 and M5 neutralized HIV-1 isolates MN and RF but not IIIB. The antisera induced by the WSN wild-type virus did not neutralize any of the HIV-1 isolates tested at the lowest serum dilution (1:20).

**Table 1**

| NEUTRALIZATION OF HIV-1 BY SERA RAISED AGAINST CHIMERIC INFLUENZA VIRUS | | | |
|---|---|---|---|
| Neutralization titer (EC₅₀) | | | |
| antisera | MN | RF | III B |
| M1 | 53 | 95 | 20 |
| M2 | 24 | 40 | 160 |
| M3 | 34 | 160 | 67 |
| M4 | 29 | 24 | not tested |
| M5 | 20 | 34 | 24 |

For syncytia inhibition assay the indicator cell line AA-2 which is described by Chaffee et al., J. Exp. Med. (1988) 168:605, and as virus inoculum frozen stocks of HIV-1 strains MN, RF and III B were used. All virus stocks were diluted to 10^{1.9}-10^{2.5} TCID₅₀ per ml. Mouse-antisera were diluted 2-fold in medium and distributed in 96-well microtiter plates (4 replicas of each dilution). 50 µl of virus was added to 50 µl of diluted antisera and the virus/antibody mixture was preincubated for 2h at 4°C. For infection, 100µl of AA-2 cells (5x10⁶ cells/ml) were added to each well; presence of syncytia was recorded after 5 days as an indication of HIV-1 infection. Estimation of 50% inhibiting dose (EC₅₀) was done according to Reed and Muench as described in Am. J. Hyg. (1938) 27:493. The reciprocal serum dilutions that inhibit syncytia formation by 50% (EC₅₀) are shown.

### Example 4: Expression of chimeric hemagglutinins carrying the extended 2F5-epitopes by recombinant baculoviruses

The chimeric hemagglutinins containing the said six peptides were prepared as described in Example 1. The coding sequence of these chimeric hemagglutinins was flanked by restriction enzyme site BamHI and inserted into the BamHI site of plasmid (Bluebac III, Invitrogen, San Diego CA). Transfection experiments in order to obtain recombinant baculoviruses that contain the chimeric hemagglutinins were done according to methods described by Groebe et al., Nucleic. Acids Res. (1990) 18:4033, and Felgner et al., Proc. Natl. Acad. Sci. USA (1987) 84:7413. The resulting recombinant chimeric baculoviruses can be used to elicit HIV-1 neutralizing antibodies.

### Example 5: Immunization and antibody response of mice immunized with cells infected by the recombinant baculoviruses

Sf9 cells that were used for immunizations, were infected by recombinant baculoviruses at a multiplicity of infection (MOI) of 1-5, and harvested 3 days post infection. The cells were washed twice with PBS and resuspended in sterile PBS at a concentration of 5x 10⁶ cells/ml. These cells specifically reacted with monoclonal antibody 2F5 in Western blots, indicating that these cells contained the hemagglutinin carrying the ELDKWAS or LELDKWAS sequence. Balb/c mice were immunized with four intraperitoneal injections of 1x10⁶ infected Sf9 cells at 2 weeks intervals (Van Wyyke Coelingh et al., Virology (1987) 160: 4465). Seven days after the fourth immunization, the mice were bled and ELISA titers of the antisera were determined. Fig.4 shows the binding of the recombinant baculovirus derived antisera to a synthetic peptide comprising the sequence Gly Gly Gly Glu Leu Asp Lys Trp Ala (GGGELDKWA) in an ELISA that was performed as described in Example 2.

### Induction of secretory antibodies

Immunizations carried out by application of at least one, preferably a mixture, of said six peptides resulted in significantly improved IgG ELDKWA-specific ELISA titers. Furthermore, in contrast to immunizations using the ELDKWA sequence, immunizations with the said six peptides resulted also in a significant IgA response. Surprisingly, this IgA immune response was also triggered at the mucosal level.

### Example 6: IgA antibodies in respiratory secretions of Balb/c mice immunized with influenza-ELDKWAS virus.

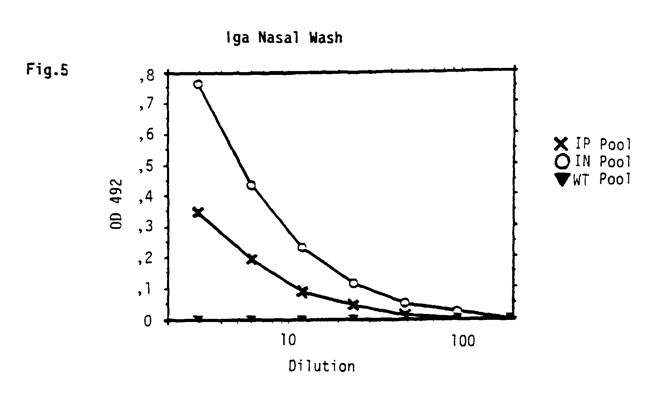

Mice were immunized with 10² PFU intranasally and boosted after 4 weeks with 10⁵ PFU by the same route. The third immunization was given intranasally (IN) or intraperitoneally (IP) with 10⁷ PFU after 4 more weeks. Nasal washings were collected and pooled 8 weeks after the third immunization and reactivity of these samples with the peptide ELDKWA was determined by ELISA. As a control (WT pool) nasal washings of mice immunized with the wild-type (WT) influenza virus were pooled and analyzed. The same immunization scheme as for the IN group was used (rf. Fig.5). The elicited antibodies have primarily been produced by the nasal mucosa and it can be recognized that the antibody titers are unusually high. It can further be seen in Fig.5 that intranasal application of the influenza-ELDKWAS-virus leads to a higher concentration of HIV-1 neutralizing antibodies than intraperitoneal application.

### Example 7: IgA antibodies in intestinal secretions of Balb/c mice immunized with influenza-ELDKWAS virus

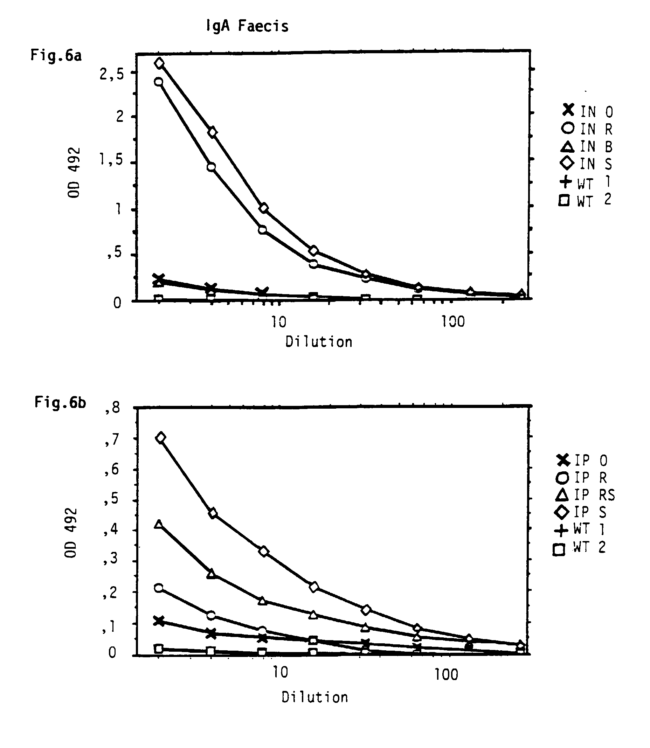

Mice were immunized with 10² PFU intranasally and boosted after 4 weeks with 10⁵ PFU by the same route. The third immunization was given intranasally (rf. Fig. 6a) or intraperitoneally (Fig. 6b) with 10⁷ PFU after 4 more weeks. Faecal pellets containing the antibodies released from the intestinal mucosa were collected and extracted 8 weeks after the third immunization, and reactivity of these samples with the peptide ELDKWA was determined by ELISA. IN O, IN R, IN B and IN S are samples from mice that received the third immunization intranasally, and the samples IP O, IP R, IP RS and IP S were collected from mice that received the third immunization intraperitoneally. WT1 and WT2 are samples of control mice immunized with wild-type (WT) influenza virus.

## Claims

1. A peptide eliciting antibodies that show neutralizing activity against different strains and/or clinical isolates of HIV-1 and/or that inhibit the fusion of cells caused by HIV-1, characterized in that the peptide is composed of an amino acid sequence selected from the group consisting of ELDKWAS, LELDKWAS, ELDNWAS, ELNKWAS, LELDNWAS and LELNKWAS.

2. A peptide according to claim 1, characterized in that it is linked to a carrier.

3. A peptide according to claim 2, characterized in that it is part of a - preferably recombinant - virus, advantageously selected from the group consisting of an influenza virus, a baculovirus and a vaccinia virus.

4. A peptide according to claim 2 or 3, characterized in that at least one of said amino acid sequences substitutes at least part of the amino acid sequence of a viral protein, or is inserted into at least one antigenic site of a viral protein.

5. A peptide according to claim 4, characterized in that the viral protein is selected from the group consisting of hemagglutinin (HA)of influenza virus, neuraminidase (NA) of influenza virus and the surface antigen of hepatitis B virus.

6. A peptide according to claim 4, characterized in that the viral protein is derived from a - preferably recombinant - baculovirus or vaccinia virus.

7. Use of at least one peptide according to anyone of claims 1 to 6 for the manufacture of a pharmaceutical to elicit anti-HIV-1 antibodies in animals and humans.

8. Use according to claim 7, wherein said antibodies are IgA antibodies, preferably secreted from mucosal surfaces.

9. Use according to claim 7 or 8 for the manufacture of a pharmaceutical for the prophylactic and/or therapeutic treatment of HIV-1 endangered or infected individuals.

10. An antibody with HIV-1 neutralizing activity and being capable of preventing fusion of cells caused by HIV-1, characterized in that it is an IgA antibody that is capable of binding to a peptide carrying the amino acid sequence ELDKWA

11. The antibody according to claim 10, secreted by a mucosal surface.

12. A process for the manufacture of a peptide according to claim 1, characterized in that an amino acid sequence as defined in claim 1 is chemically synthesized, preferably by means of a commercial peptide synthesizer.

13. A process for the manufacture of a peptide according to claim 1, characterized in that the peptide is produced by way of a microbiological method, said method comprising inserting a nucleotide sequence selected from the group consisting of
a) a nucleotide sequence corresponding to one of said amino acid sequences;
b) a nucleotide sequence hybridizing to one of the nucleotide sequences under a);
c) a nucleotide sequence deduced from one of the nucleotide sequences under a) by degeneration;
into the genome of a host organism, carrying out expression of the genome using standard microbiological cultivation techniques, and isolating at least one, preferably a multitude, of said peptides.

14. A process for the manufacture of at least one peptide according to anyone of claims 2 to 6, comprising linking an amino acid sequence of claim 1 to a carrier, preferably a virus or viral protein, by chemical or microbiological methods.

15. The process according to claim 14 wherein said method is microbiological and comprises the steps of linking a nucleotide sequence selected from the group consisting of
a) a nucleotide sequence corresponding to one of said amino acid sequences;
b) a nucleotide sequence hybridizing to one of the nucleotide sequences under a);
c) a nucleotide sequence deduced from one of the nucleotide sequences under a) by degeneration;
to a nucleotide sequence corresponding to an amino acid sequence of the carrier, transferring the linked nucleotide sequences to a host organism, carrying out expression of the linked sequence using standard microbiological techniques, and isolating at least one, preferably a multitude, of peptides linked to said carrier.

16. A process according to claim 15, wherein at least one of the nucleotide sequences a) through c) is linked to a nucleotide sequence corresponding to an amino acid sequence of a viral protein as the carrier, thereby substituting at least part of the nucleotide sequence corresponding to the amino acid sequence of the viral protein or being inserted into at least one site of said sequence corresponding to an antigenic site of the viral protein.

17. A process according to claim 16, wherein said viral protein is selected from the group consisting of the hemagglutinin of influenza virus, neuraminidase of influenza virus and the surface antigen of hepatitis B virus.

18. A process according to anyone of claims 13, 15 to 17, wherein a virus of the group consisting of a - preferably recombinant - influenza virus, baculovirus and vaccinia virus is used as the host organism.

19. A process for the production of an antibody showing HIV-1 neutralizing activity and being capable of preventing fusion of cells caused by HIV-1, characterized in that a peptide selected from the group consisting of ELDKWAS, LELDKWAS, ELDNWAS, ELNKWAS, LELDNWAS and LELNKWAS, said peptide preferably being linked to a carrier, is applied to the immune system of a human or animal, whereupon said antibody is produced.

20. The process according to claim 19, wherein said peptide is applied to the immune system via a mucosal surface, preferably intranasally.

21. The process according to claim 19 or 20, wherein said antibody is produced via secretion by a mucosal surface.

22. The process according to anyone of claims 19 to 21 with said peptide linked to a carrier, wherein said carrier is a virus, preferably influenza virus, or part of a virus, preferably hemagglutinin of influenza virus.

23. The process according to anyone of claims 19 to 22, wherein the produced antibody is an IgA antibody.

24. An anti-HIV-1 vaccine comprising at least one peptide, preferably a mixture of at least two peptides according to anyone of claims 1 to 6.

## Patentansprüche

1. Peptid, Antikörper induzierend, die eine neutralisierende Aktivität gegenüber verschiedenen Stämmen und/oder klinischen Isolaten von HIV-1 aufweisen und/oder die von HIV-1 verursachte Zellfusion inhibieren, dadurch gekennzeichnet, dass das Peptid aus einer aus der aus ELDKWAS, LELDKWAS, ELDNWAS, ELNKWAS, LELDNWAS und LELNKWAS bestehenden Gruppe ausgewählten Aminosäuresequenz zusammengesetzt ist.

2. Peptid nach Anspruch 1, dadurch gekennzeichnet, dass es mit einem Träger verbunden ist.

3. Peptid nach Anspruch 2, dadurch gekennzeichnet, dass es Teil eines - bevorzugt rekombinanten - Virus ist, der vorteilhaft aus der aus einem Influenzavirus, einem Baculovirus und einem Vacciniavirus bestehenden Gruppe ausgewählt ist.

4. Peptid nach Anspruch 2 oder 3, dadurch gekennzeichnet, dass zumindest eine der benannten Aminosäuresequenzen zumindest einen Teil der Aminosäuresequenz eines Virusproteins ersetzt oder in zumindest einer Antigenerkennungsstelle eines Virusproteins eingesetzt ist.

5. Peptid nach Anspruch 4, dadurch gekennzeichnet, dass das Virusprotein aus der Gruppe ausgewählt ist, die aus Hämagglutinin (HA) des Influenzavirus, Neuraminidase (NA) des Influenzavirus und dem Oberflächenantigen des Hepatitis-B-Virus besteht.

6. Peptid nach Anspruch 4, dadurch gekennzeichnet, dass das Virusprotein von einem - bevorzugt rekombinanten - Baculovirus oder Vacciniavirus abgeleitet ist.

7. Verwendung von zumindest einem Peptid nach einem der Ansprüche 1 bis 6 zur Herstellung eines Medikaments zur Induktion von Anti-HIV-1-Antikörpern in Menschen und Tieren.

8. Verwendung nach Anspruch 7, worin die benannten Antikörper bevorzugt aus Schleimhautoberflächen ausgeschiedene IgA-Antikörper sind.

9. Verwendung nach Anspruch 7 oder 8 für die Herstellung eines Medikaments für die prophylaktische und/oder therapeutische Behandlung von HIV-1-gefährdeten oder -infizierten Individuen.

10. Antikörper mit HIV-1-neutralisierender Aktivität und in der Lage, die durch HIV-1 verursachte Zellfusion zu verhindern, dadurch gekennzeichnet, dass er ein IgA-Antikörper ist, der in der Lage ist, an ein die Aminosäuresequenz ELDKWA tragendes Peptid zu binden.

11. Antikörper nach Anspruch 10, von einer Schleimhautoberfläche ausgeschieden.

12. Verfahren zur Herstellung eines Peptids nach Anspruch 1, dadurch gekennzeichnet, dass eine wie in Anspruch 1 definierte Aminosäuresequenz chemisch synthetisiert wird, vorzugsweise mittels eines handelsüblichen Peptidsynthetisators.

13. Verfahren zur Herstellung eines Peptis nach Anspruch 1, dadurch gekennzeichnet, dass das Peptid durch eine mikrobiologische Methode hergestellt wird, wobei die benannte Methode umfasst, in das Genom eines Wirtsorganismus eine Nucleotidsequenz einzusetzen, die aus der Gruppe ausgewählt ist, die aus
a) einer Nucleotidsequenz, die einer der benannten Aminosäuresequenzen entspricht;
b) einer Nucleotidsequenz, die zu einer der Nucleotidsequenzen unter (a) hybridisiert;
c) einer durch Degeneration aus einer der Nucleotidsequenzen unter (a) abgeleiteten Nucleotidsequenz
besteht; die Expression des Genoms unter Verwendung mikrobiologischer Standard-Kultivierungsverfahren auszuführen und zumindest eines, vorzugsweise eine Vielzahl der benannten Peptide zu isolieren.

14. Verfahren zur Herstellung von zumindest einem Peptid nach einem der Ansprüche 2 bis 6, einschliessend, eine Aminosäuresequenz des Anspruchs 1 durch chemische oder mikrobiologische Methoden mit einem Träger, vorzugsweise einem Virus oder einem Virusprotein, zu verbinden.

15. Verfahren nach Anspruch 14, worin die benannte Methode mikrobiologisch ist und die Schritte umfasst, mit einer einer Aminosäuresequenz des Trägers entsprechenden Nucleotidsequenz eine Nucleotidsequenz zu verbinden, die aus der Gruppe ausgewählt ist, die aus
a) einer Nucleotidsequenz, die einer der benannten Aminosäuresequenzen entspricht;
b) einer Nucleotidsequenz, die zu einer der Nucleotidsequenzen unter (a) hybridisiert;
c) einer durch Degeneration aus einer der Nucleotidsequenzen unter (a) abgeleiteten Nucleotidsequenz
besteht; die verbundenen Nucleotidsequenzen zu einem Wirtsorganismus zu überführen, die Expression der verbundenen Sequenz unter Verwendung mikrobiologischer Standard-Kultivierungsverfahren auszuführen und zumindest eines, vorzugsweise eine Vielzahl der mit dem benannten Träger verbundenen Peptide zu isolieren.

16. Verfahren nach Anspruch 15, worin zumindest eine der Nucleotidsequenzen (a) bis (c) mit einer Nucleotidsequenz verbunden ist, die einer Aminosäuresequenz eines Virusproteins als des Trägers entspricht, wodurch zumindest ein Teil der Nucleotidsequenz ersetzt wird, die der Aminosäuresequenz des Virusproteins entspricht oder an zumindest einer Stelle der benannten Sequenz eingesetzt wird, die einer Antigenerkennungsstelle des Virusproteins entspricht.

17. Verfahren nach Anspruch 16, worin das benannte Virusprotein aus der Gruppe ausgewählt ist, die aus Hämagglutinin des Influenzavirus, Neuraminidase des Influenzavirus und dem Oberflächenantigen des Hepatitis-B-Virus besteht.

18. Verfahren nach einem der Ansprüche 13, 15 bis 17, worin ein Virus der Gruppe, die aus einem - bevorzugt rekombinanten - Influenzavirus, Baculovirus und Vacciniavirus besteht, als Wirtsorganismus verwendet wird.

19. Verfahren zur Herstellung eines Antikörpers mit HIV-1 neutralisierender Aktivität und in der Lage, die durch HIV-1 verursache Zellfusion zu verhindern, dadurch gekennzeichnet, dass auf das Immunsystem eines Menschen oder Tieres ein bevorzugt mit einem Träger verbundenes Peptid angesetzt wird, das aus der aus ELDKWAS, LELDKWAS, ELDNWAS, ELNKWAS, LELDNWAS und LELNKWAS bestehenden Gruppe ausgewählt ist, woraufhin der benannte Antikörper erzeugt wird.

20. Verfahren nach Anspruch 19, worin das benannte Peptid über eine Schleimhautoberfläche, bevorzugt intranasal, auf das Immunsystem angesetzt wird.

21. Verfahren nach Anspruch 19 oder 20, worin der benannte Antikörper über Ausscheidung durch eine Schleimhautoberfläche erzeugt wird.

22. Verfahren nach einem der Ansprüche 19 bis 21, wobei das benannte Peptid mit einem Träger verbunden ist und worin der benannte Träger ein Virus, vorzugsweise ein Influenzavirus, oder ein Teil eines Virus, vorzugsweise Hämagglutinin des Influenzavirus, ist.

23. Verfahren nach einem der Ansprüche 19 bis 22, worin der erzeugte Antikörper ein IgA-Antikörper ist.

24. Anti-HIV-1-Impfstoff mit zumindest einem Peptid, vorzugsweise einer Mischung von zumindest zwei Peptiden nach einem der Ansprüche 1 bis 6.

## Revendications

1. Peptide produisant des anticorps qui présentent une activité neutralisante contre des souches différentes et/ou des isolats cliniques différents de VIH-1 et/ou qui inhibent la fusion des cellules provoquée par le VIH-1, caractérisé en ce que le peptide est composé d'une séquence d'acides aminés choisie parmi le groupe constitué de ELDKWAS, LELDKWAS, ELDNWAS, ELNKWAS, LELDNWAS et LELNKWAS.

2. Peptide selon la revendication 1, caractérisé en ce qu'il est lié à un vecteur.

3. Peptide selon la revendication 2, caractérisé en ce qu'il fait partie d'un virus - de préférence recombinant -, choisi de façon avantageuse parmi le groupe constitué d'un virus de la grippe, d'un baculovirus et d'un virus de la vaccine.

4. Peptide selon la revendication 2 ou 3, caractérisé en ce qu'au moins l'une desdites séquences d'acides aminés remplace au moins une partie de la séquence d'acides aminés d'une protéine virale, ou bien est insérée dans au moins un site antigénique d'une protéine virale.

5. Peptide selon la revendication 4, caractérisé en ce que la protéine virale est choisie parmi le groupe constitué de l'hémagglutinine (HA) du virus de la grippe, de la neuraminidase (NA) du virus de la grippe et de l'antigène d'enveloppe du virus de l'hépatite B.

6. Peptide selon la revendication 4, caractérisé en ce que la protéine virale est obtenue à partir d'un baculovirus ou d'un virus de la vaccine - de préférence recombinant -.

7. Utilisation d'au moins un peptide conforme à l'une quelconque des revendications 1 à 6 en vue de la fabrication d'un produit pharmaceutique pour produire des anticorps anti-VIH-1 chez les animaux et les humains.

8. Utilisation selon la revendication 7, dans laquelle lesdits anticorps sont des anticorps IgA, sécrétés de préférence paf des surfaces muqueuses.

9. Utilisation selon la revendication 7 ou 8, en vue de la fabrication d'un produit pharmaceutique pour le traitement prophylactique et/ou thérapeutique d'individus exposés ou contaminés par le VIH-1.

10. Anticorps présentant une activité de neutralisation du VIH-1 et étant capable d'empêcher la fusion des cellules provoquée par le VIH-1, caractérisé en ce qu'il s'agit d'un anticorps IgA qui est capable de se lier à un peptide portant la séquence d'acides aminés ELDKWA.

11. Anticorps selon la revendication 10, sécrété par une surface muqueuse.

12. Procédé destiné à la fabrication d'un peptide conforme à la revendication 1, caractérisé en ce qu'une séquence d'acides aminés telle que définie dans la revendication 1 est synthétisée chimiquement, de préférence au moyen d'un agent de synthèse de peptides du commerce.

13. Procédé destiné à la fabrication d'un peptide conforme à la revendication 1, caractérisé en ce que le peptide est produit au moyen d'un procédé microbiologique, ledit procédé comprenant l'insertion d'une séquence nucléotidique choisie parmi le groupe constitué de
a) une séquence nucléotidique correspondant à l'une desdites séquences d'acides aminés,
b) une séquence nucléotidique s'hybridant à l'une des séquences nucléotidiques selon a),
c) une séquence nucléotidique issue de l'une des séquences nucléotidiques selon a) par dégénérescence,
dans le génome d'un organisme hôte, l'exécution de l'expression du génome en utilisant des techniques de culture microbiologiques standard, et l'isolement d'au moins l'un, de préférence une multitude, desdits peptides.

14. procédé destiné à la fabrication d'au moins un peptide conforme à l'une quelconque des revendications 2 à 6, comprenant la liaison d'une séquence d'acides aminés de la revendication 1 à un vecteur, de préférence un virus ou une protéine virale, par des procédés chimiques ou microbiologiques.

15. Procédé selon la revendication 14, dans lequel ledit procédé est microbiologique et comprend les étapes consistant à lier une séquence nucléotidique choisie parmi le groupe constitué de
a) une séquence nucléotidique correspondant à l'une desdites séquences d'acides aminés,
b) une séquence nucléotidique s'hybridant à l'une des séquences nucléotidiques selon a),
c) une séquence nucléotidique issue de l'une des séquences nucléotidiques selon a) par dégénérescence,
à une séquence nucléotidique correspondant à une séquence d'acides aminés du vecteur, transférer les séquences nucléotidiques liées à un organisme hôte, exécuter l'expression de la séquence liée en utilisant des techniques microbiologiques standard, et isoler au moins l'un, de préférence une multitude, des peptides liés audit vecteur.

16. Procédé selon la revendication 15, dans lequel au moins l'une des séquences nucléotidiques a) à c) est liée à une séquence nucléotidique correspondant à une séquence d'acides aminés d'une protéine virale en tant que vecteur, en remplaçant ainsi au moins une partie de la séquence nucléotidique correspondant à la séquence d'acides aminés de la protéine virale ou bien en étant insérée dans au moins un site de ladite séquence correspondant à un site antigénique de la protéine virale.

17. Procédé selon la revendication 16, dans lequel ladite protéine virale est choisie parmi le groupe constitué de l'hémagglutinine du virus de la grippe, de la neuraminidase du virus de la grippe et de l'antigène d'enveloppe du virus de l'hépatite B.

18. Procédé selon l'une quelconque des revendications 13, 15 à 17, dans lequel un virus du groupe constitué d'un virus de la grippe, d'un baculovirus et d'un virus de la vaccine - de préférence recombinants -, est utilisé en tant qu'organisme hôte.

19. Procédé destiné à la production d'un anticorps présentant une activité de neutralisation du VIH-1 et étant capable d'empêcher la fusion des cellules provoquée par le VIH-1, caractérisé en ce qu'un peptide choisi parmi le groupe constitué de ELDKWAS, LELDKWAS, ELDNWAS, ELNKWAS, LELDNWAS, et LELNKWAS, ledit peptide étant de préférence lié à un vecteur, est appliqué au système immunitaire d'un humain ou d'un animal, à la suite de quoi ledit anticorps est produit.

20. Procédé selon la revendication 19, dans lequel ledit peptide est appliqué au système immunitaire par l'intermédiaire d'une surface muqueuse, de préférence de manière intranasale.

21. Procédé selon la revendication 19 ou 20, dans lequel ledit anticorps est produit par l'intermédiaire d'une sécrétion par une surface muqueuse.

22. Procédé selon l'une quelconque des revendications 19 à 21, ledit peptide étant lié à un vecteur, dans lequel ledit vecteur est un virus, de préférence le virus de la grippe, ou une partie d'un virus, de préférence l'hémagglutinine du virus de la grippe.

23. Procédé selon l'une quelconque des revendications 19 à 22, dans lequel l'anticorps produit est un anticorps IgA.

24. Vaccin anti-VIH-1 comprenant au moins un peptide, de préférence un mélange d'au moins deux peptides conformes à l'une quelconque des revendications 1 à 6.
